# FASCICULE DE BREVET EUROPEEN

(11) **EP 0 854 699 B1**
(45) Date de publication et mention de la délivrance du brevet: **28.07.2004**
(21) Numéro de dépôt: 96931854.2
(22) Date de dépôt: 16.09.1996
(51) Int. Cl.: A61K 7/06, A61K 7/48

(54) **COMPOSITION TOPIQUE CONTENANT UN POLYMERE SILICONE A SQUELETTE POLYSILOXANE A GREFFONS NON-SILICONES ET UN POLYMERE AMPHIPHILE A CHAINE GRASSE**
ZUSAMMNENSETZUNG ZUR TOPISCHEN ANWENDUNG, DIE EIN SILICONPOLYMER MIT POLYSILOXANGERÜST UND NICHT SILIKONHALTIGEN PFROPFZWEIGEN UND EIN AMPHIPHILES POLYMER MIT FETTKETTE ENTHÄLT
TOPICAL COMPOSITION CONTAINING A SILICONE POLYMER WITH A POLYSILOXANE BACKBONE HAVING NON-SILICONE GRAFTS AND A FATTY-CHAIN AMPHIPHILIC POLYMER

(30) Priorité: 29.09.1995 FR 9511482
(43) Date de publication de la demande: 29.07.1998
(73) Titulaire: L'OREAL, 75008 Paris (FR)
(72) Inventeur: DUBIEF, Claude, F-78150 Le Chesnay (FR); DUPUIS, Christine, F-75018 Paris (FR)
(74) Mandataire: Le Blainvaux Bellegarde, Françoise
(86) Numéro de dépôt international: PCT/FR1996/001436
(87) Numéro de publication internationale: WO 1997/012586

(56) Documents cités:
- EP-A- 0 412 705
- EP-A- 0 524 612
- EP-A- 0 582 152
- WO-A-91/15186
- WO-A-92/16179
- WO-A-95/00108
- WO-A-95/05800
- WO-A-95/06078
- FR-A- 2 709 955

## Description

La présente invention a trait à une composition cosmétique ou dermatologique pour le traitement des matières kératiniques, en particulier des cheveux humains, comprenant au moins un polymère siliconé greffé, à squelette polysiloxanique greffé par des monomères organiques non-siliconés et au moins un polymère amphiphile comportant au moins une chaîne grasse et au moins un motif hydrophile.

Les polymères du type polymère siliconé greffé, à squelette polysiloxanique greffé par des monomères organiques non-siliconés sont connus dans l'art antérieur pour leurs propriétés coiffantes. Ils sont particulièrement intéressants en cosmétique capillaire du fait qu'ils apportent de la tenue aux cheveux. Leurs propriétés cosmétiques après application sur les cheveux sont néanmoins insuffisantes. On constate que les cheveux présentent après application de ces polymères, un toucher rêche et crissant résultant d'une répartition discontinue du polymère le long des fibres des cheveux.

La demanderesse a constaté que certains agents épaississants classiques, tels que, par exemple les homopolymères poly(acide acrylique) réticulés, utilisés dans des composions capillaires contenant ces polymères particuliers. avaient tendance à diminuer la viscosité de la composition et ne permettaient pas d'améliorer sensiblement la répartition de la composition le long des fibres de cheveux mouillés ou séchés ni d'améliorer sensiblement les propriétés de douceur au toucher ou de démêlage, après application.

La demanderesse a trouvé de façon surprenante que l'utilisation d'un polymère amphiphile comportant au moins une chaîne grasse et au moins un motif hydrophile, comme agent épaississant dans des compositions capillaires contenant un polymère à squelette polysiloxanique greffé par des monomères organiques non-siliconés, permettait non seulement d'augmenter de façon accrue la viscosité du milieu de ces compositions mais aussi d'améliorer, à l'application, le dépôt du polymère siliconé greffé le long des fibres kératiniques et d'améliorer leurs propriétés cosmétiques notamment au niveau du toucher ainsi qu'au niveau du démêlage, tout en conservant les propriétés coiffantes du polymère siliconé greffé.

La composition selon l'invention est donc essentiellement caractérisée par le fait qu'elle contient dans un milieu cosmétiquement ou dermatologiquement acceptable au moins un polymère siliconé greffé, à squelette polysiloxanique greffé par des monomères organiques non-siliconés et au moins un polymère amphiphile comportant au moins une chaîne grasse et au moins un motif hydrophile.

Dans ce qui suit, on entend désigner par polymère siliconé, en conformité avec l'acceptation générale, tous polymères ou oligomères organosiliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyls notamment en C₁-C₁₀ et en particulier méthyle, les radicaux fluoroalkyls, les radicaux aryls et en particulier phényle, et les radicaux alcényls et en particulier vinyle ; d'autres types de radicaux susceptibles d'être liés soit directement, soit par l'intermédiaire d'un radical hydrocarboné, à la chaîne siloxanique sont notamment l'hydrogène, les halogènes et en particulier le chlore, le brome ou le fluor, les thiols, les radicaux alcoxy, les radicaux polyoxyalkylènes (ou polyéthers) et en particulier polyoxyéthylène et/ou polyoxypropylène, les radicaux hydroxyls ou hydroxyalkyls, les groupements aminés substitués ou non. les groupements amides, les radicaux acyloxy ou acyloxyalkyls, les radicaux hydroxyalkylamino ou aminoalkyls, des groupements ammonium quaternaires. des groupements amphotères ou bétaïniques, des groupements anioniques tels que carboxylates, thioglycolates, sulfosuccinates, thiosulfates, phosphates et sulfates, cette liste n'étant bien entendu nullement limitative (silicones dites "organomodifiées").

Selon la présente invention, le ou les polymères siliconés qui doivent être utilisés sont ceux qui comprennent une chaîne principale de silicone (ou polysiloxane (=Si-O-)ₙ) sur laquelle se trouve greffé, à l'intérieur de ladite chaîne ainsi qu'éventuellement à l'une au moins de ses extrémités, au moins un groupement organique ne comportant pas de silicone.

Ces polymères siliconés peuvent être des produits commerciaux existants, ou encore être obtenus selon tout moyen connu de l'homme de l'art, en particulier par réaction entre (i) une silicone de départ correctement fonctionnalisée sur un ou plusieurs de ces atomes de silicium et (ii) un composé organique non-siliconé lui-même correctement fonctionnalisé par une fonction qui est capable de venir réagir avec le ou les groupements fonctionnels portés par ladite silicone en formant une liaison covalente ; un exemple classique d'une telle réaction est la réaction d'hydrosylilation entre des groupements ≡Si-H et des groupements vinyliques CH₂=CH-, ou encore la réaction entre des groupements thio-fonctionnels -SH avec ces mêmes groupements vinyliques.

Des exemples de polymères siliconés convenant à la mise en oeuvre de la présente invention, ainsi que leur mode particulier de préparation, sont notamment décrits dans les demandes de brevets EP-A-0582152, WO93/23009 et WO 95/03776 dont les enseignements sont totalement inclus dans la présente description à titre de références non limitatives.

Selon un mode particulièrement préféré de réalisation de la présente invention, le polymère siliconé mis en oeuvre comprend le résultat de la copolymérisation radicalaire entre d'une part au moins un monomère organique anionique non-siliconé présentant une insaturation éthylénique et/ou un monomère organique hydrophobe non-siliconé présentant une insaturation éthylénique et d'autre part une silicone présentant dans sa chaîne au moins un groupement fonctionnel capable de venir réagir sur lesdites insaturations éthyléniques desdits monomères non-siliconés en formant une liaison covalente, en particulier des groupements thio-fonctionnels.

Selon la présente invention, lesdits monomères anioniques à insaturation éthylénique sont de préférence choisis, seuls ou en mélanges, parmi les acides carboxyliques insaturés, linéaires ou ramifiés, éventuellement partiellement ou totalement neutralisés sous la forme d'un sel, ce ou ces acides carboxyliques insaturés pouvant être plus particulièrement l'acide acrylique, l'acide méthacrylique, l'acide maléique, l'anhydride maléique, l'acide itaconique, l'acide fumarique et l'acide crotonique. Les sels convenables sont notamment les sels d'alcalins, d'alcalino-terreux et d'ammonium. On notera que, de même, dans le polymère siliconé greffé final, le groupement organique à caractère anionique qui comprend le résultat de l'(homo)polymérisation radicalaire d'au moins un monomère anionique de type acide carboxylique insaturé peut être, après réaction, post-neutralisé avec une base (soude, ammoniaque,...) pour l'amener sous la forme d'un sel.

Selon la présente invention, les monomères hydrophobes à insaturation éthylénique sont de préférence choisis, seuls ou en mélanges, parmi les esters d'acide acrylique alcanols et/ou les esters d'acide méthacrylique d'alcanols. Les alcanols sont de préférence en C₁-C₁₈ et plus particulièrement en C₁-C₁₂. Les monomères préférentiels sont choisis dans le groupe constitué par le (méth)acrylate d'isooctyle, le (méth) acrylate d'isononyle, le 2-éthylhexyl(méth)acrylate, le (méth)acrylate de lauryle, le (méth)acrylate d'isopentyle, le (méth)acrylate de n-butyle, le (méth)acrylate d'isobutyle, le (méth)acrylate de méthyle, le (méth)acrylate de tertio-butyle, le (méth) acrylate de tridécyle, le (méth)acrylate de stéaryle ou leurs mélanges.

Une famille de polymères siliconés greffés particulièrement bien à la mise en oeuvre de la présente invention est constituée par les polymères siliconés comportant dans leur structure le motif de formule (I) suivant : 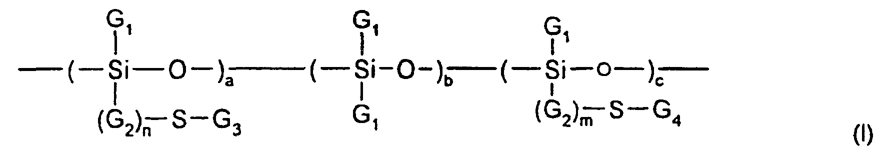 dans lequel les radicaux G₁, identiques ou différents, représentent l'hydrogène ou un radical alkyle en C₁-C₁₀ ou encore un radical phényle ; les radicaux G₂, identiques ou différents, représentent un groupe alkylène en C₁-C₁₀ ; G₃ représente un reste polymérique résultant de l'(homo)polymérisation d'au moins un monomère anionique à insaturation éthylénique ; G₄ représente un reste polymérique résultant de l'(homo) polymérisation d'au moins un monomère hydrophobe à insaturation éthylènique ; m et n sont égaux à 0 ou 1 ; a est un nombre entier allant de 0 et 50 ; b est un nombre entier pouvant être compris entre 10 et 350, c est un nombre entier allant de 0 et 50 ; sous réserve que l'un des paramètres a et c soit différent de 0.

De préférence, le motif de formule (I) ci-dessus présente au moins l'une, et encore plus préférentiellement l'ensemble, des caractéristiques suivantes :
- les radicaux G₁ désignent un radical alkyle, de préférence le radical méthyle ;
- n est non nul, et les radicaux G₂ représentent un radical divalent en C₁-C₃, de préférence un radical propylène ;
- G₃ représente un radical polymérique résultant de l'(homo)polymérisation d'au moins un monomère du type acide carboxylique à insaturation éthylénique, de préférence l'acide acrylique et/ou l'acide méthacrylique ;
- G₄ représente un radical polymérique résultant de l'(homo)polymérisation d'au moins un monomère du type (méth)acrylate d'alkyle (C₁-C₁₀), de préférence le (méth)acrylate d'isobutyle ou de méthyle.

Des exemples de polymères siliconés répondant à la formule (I) sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate de méthyle.

D'autres exemples de polymères siliconés répondant à la formule (I) sont notamment des polydiméthylsiloxanes (PDMS) sur lesquels sont greffés, par l'intermédiaire d'un chaînon de raccordement de type thiopropylène, des motifs polymères du type poly(méth)acrylate d'isobutyle.

De préférence, la masse moléculaire en nombre des polymères siliconés de l'invention varie de 10 000 à 1 000 000 environ, et encore plus préférentiellement de 10 000 à 100 000 environ.

Le polymère siliconé greffé est utilisé de préférence en une quantité allant de 0,01 à 20% en poids du poids total de la composition. Plus préférentiellement, cette quantité varie de 0,1 à 15% en poids et encore plus préférentiellement de 0,5 à 10% en poids.

Les polymères amphiphiles comportant au moins une chaîne grasse et au moins un motif hydrophile, utilisés selon l'invention sont choisis de préférence dans le groupe constitué par :
(1) les holosides modifiés par des groupements comportant au moins une chaîne grasse ;
   on peut citer à titre d'exemple :
      - les celluloses ou leurs dérivés, modifiés par des groupements comportant au moins une chaîne grasse tels que des groupes alkyls, arylalkyls, alkylaryls ou leurs mélanges où les groupes alkyls sont en C₈-C₂₂ ;
      - les alkylhydroxyéthylcelluloses non-ioniques telles que les produits NATROSOL PLUS GRADE 330 CS et POLYSURF 67 (alkyle en C₁₆) vendus par la société AQUALON ;
      - les alkylhydroxyéthylcelluloses quatemisées (cationiques) telles que les produits QUATRISOFT LM 200, QUATRISOFT LM-X 529-18-A, QUATRISOFT LM-X 529-18-B (alkyle en C₁₂) et QUATRISOFT LM-X 529-8 (alkyle en C₁₈) vendus par la société AMERCHOL et les produits CRODACEL QM , CRODACEL QL (alkyle en C₁₂) et CRODACEL QS (alkyle en C₁₈) vendus par la société CRODA ;
      - les nonoxynylhydroxyéthylcelluloses non-ioniques tels que le produit AMERCELL HM-1500 vendu par la société AMERCHOL ;
      - les alkylcelluloses non-ioniques telles que le produit BERMOCOLL EHM 100 vendu par la société BEROL NOBEL;
      - les polyalcools(C₁₂-C₁₈) sacchandes tels que le produit EMULSAN (mélange D-galactosamine/acide aminouronique) et le produit BIOSAN LPS-50 vendus par la société PETROFERM ;
      - les hydroxypropylguars modifiés par une chaîne grasse tel que le produit ESAFLOR HM 22 (modifié par une chaîne alkyle en C₂₂) vendu par la société LAMBERTI ; le produit MIRACARE XC 95-3 (modifié par une chaîne alkyle en C₁₄) et le produit RE 205-146 (modifié par une chaîne alkyle en C₂₀) vendus par RHONE-POULENC ;
(2) les copolymères d'anhydride maléique ou de l'un de ses dérivés et de monomères comportant au moins une chaîne grasse:
   on peut citer à titre d'exemple :
      - les copolymères N-octadécylvinyléther/anhydride maléique comme le produit GANTREZ AN-8194 vendu par la société ISP ;
      - les terpolymères acétate de vinyle/monomaléate d'isobutyle/néodécanoate de vinyle tels que les produits ACV-4033 et 9649-147 vendus par ISP, le produit MEYPRO-FIX 509 vendu par MEYHALL et les produits DENSODRIN BA et LlPODERM LIQUOR FP vendus par BASF;
(3) les polyuréthanes et leurs dérivés comportant des groupements contenant au moins une chaîne grasse tels que par exemple les produits commerciaux suivants: RHEOLATE 205, 208 et 210 vendus par la société RHEOX ; BERMODOL PUR 2130 vendu par la société BEROL NOBEL; ACRYSOL SCT-275, ACRYSOL RM-870 et ACRYSOL44, DW-1206 B, DW-1206 F, DW-1206 G et DW-1206 J vendus par la société ROHM & HAAS : DAPRAL T 212 vendu par la société AKZO
(4) les copolymères de l'acide crotonique et de monomères comportant au moins une chaîne grasse ;
   on peut citer à titre d'exemple :
      - les terpolymères acétate de vinyle/acide crotonique/stéarate d'allyle ;
(5) les copolymères de N-vinylpyrrolidone et de monomères comportant au moins une chaîne grasse tels que des oléfines substituées par un radical alkyle comportant une longue chaîne hydrocarbonée comme par exemple les produits ANTARON V216 et ANTARON V220 vendus par la société ISP ;
(6) les copolymères d'acide (méth)acrylique et de monomères comportant au moins une chaîne grasse ; ces monomères sont choisis parmi les monomères hydrophobes à chaîne grasse, les monomères amphiphiles comportant une partie hydrophobe à chaîne grasse et une partie hydrophile ou bien leurs mélanges;
   on peut citer à titre d'exemples :
      - les copolymères réticulés d'acide acrylique/acrylate d'alkyle en C₁₀-C₃₀ tels que les produits PEMULEN TR 1, PEMULEN TR 2, CARBOPOL 1382, CARBOPOL 1342 et CARBOPOL ETD 2020 vendus par la société GOODRICH ;
      - les copolymères acide (méth)acrylique/acrylate d'éthyte/acrylate d'alkyle tels que le produit ACUSOL 823 vendu par la société ROHM & HAAS et le produit IMPERON R vendu par la société HOECHST ;
      - les copolymères réticulés acide acrylique/isodécanoate de vinyle tel que le produit STABYLEN 30 vendu par la société 3V;
      - les terpolymères acide acrylique/vinylpyrrolidone/méthacrylate de lauryle tels que les produits ACRYLIDONE LM, ACP-1184, ACP-1194 vendus par la société ISP ;
      - les copolymères acide acrylique/(méth)acrylate de lauryle tels que les produits COATEX SX vendus par la société COATEX ;
      - les terpolymères acide (méth)acrylique/acrylate d'alkyle/alkyl polyéthoxylé allyl éther tels que les produits RHEOVIS -CR, -CR₃, -CR₂ et -CRX vendus par la société ALLIED COLLOIDS;
      - les terpolymères acide méthacrylique/acrylate d'éthyle/stéaryl polyéthoxylé allyl éther tels que les produits SALCAR E-SC90 et-SC80 vendus par la société ALLIED COLLOIDS (stéaryl polyéthoxylé à 10 moles d'oxyde d'éthylène noté stéareth-10) ;
      - les terpolymères acide méthacrylique/acrylate d'éthyle/acrylate de lauryle polyoxyéthyléné tel que le produit RHEO 2000 vendu par COATEX ;
      - les terpolymères acide méthacryliquelacrylate d'éthyle/méthacrylate de stéaryle polyoxyéthyléné tels que les produits ACRYSOL 22, ACRYSOL 25 et DW-1206A vendus par la société ROHM & HAAS ;
      - les copolymères acide méthacrylique/acrylate d'éthylelacrylate de nonylphénol polyoxyéthyléné tels que le produit RHEO 3000 vendu par COATEX ;
      - les copolymères acide acrylique/monoitaconate de stéaryle polyoxyéthyléné ou les copolymères acide acrylique/monoitaconate de cétyle polyoxyéthyléné tels que les produits 8069-72A et 8069-72B vendus par NATIONAL STARO. 1 ;
      - les copolymères acide méthacrylique/acrylate de butyle/monomère hydrophobe comportant au moins une chaîne grasse tels que le produit 8069-146A vendu par NATIONAL STARCH ;
      - les terpolymères acide acrylique/acrylate d'alkyle en C₁₅/acrylate de polyéthylèneglycol (28 moles d'oxyde d'éthylène) tels que le produit DAPRAL GE 202 vendu par la société AKZO ;
      - les sels d'un ester d'acide gras partiel d'un copolymère acide acrylique/diméthyléthanolamine tels que le produit DAPRAL GE 202 DMA vendu par la société AKZO ;
      - les copolymères acide acrylique/acrylate/monomère amphiphile comportant une chaîne grasse à groupements uréthane tels que le produit ADDITOL VXW 1312 vendu par HOECHST ;
      - les copolymères acryliques modifiés par des groupes hydrophobes à chaîne grasse tels que le produit ACUSOL 102 vendu par ROHM & HAAS ;
(7) les copolymères non-ioniques de (méth)acrylate d'alkyle inférieur (C₁-C₆) et de monomères amphiphiles comportant une chaîne grasse comme par exemple les copolymères de méthacrylate de méthylelacrylate de stéaryle polyoxyéthyléné tel que le produit ANTIL 208 vendu par la société GOLDSCHMIDT ;
(8) les copolymères non-ioniques de (méth)acrylates hydrophiles et de monomères hydrophobes à chaîne grasse comme par exemple les copolymères méthacrylate de polyéthylèneglycol/méthacrylate de méthyle.

Les polymères amphiphiles comportant au moins une chaîne grasse et des motifs hydrophiles selon l'invention, sont utilisés de préférence en une quantité comprise entre 0,01 et 20% en poids du poids total de la composition. Plus préférentiellement, cette quantité varie de 0,1 à 15% en poids et encore plus préférentiellement de 0,5 à 10% en poids.

Le milieu cosmétiquement ou dermatologiquement acceptable est de préférence constitué par de l'eau ou un mélange d'eau et de solvants cosmétiquement acceptables tels que des monoalcools, des polyalcools, des éthers de glycol ou des esters d'acides gras, qui peuvent être utilisés seuls ou en mélange.

On peut citer plus particulièrement les alcools inférieurs tels que l'éthanol, l'isopropanol, les polyalcools tels que le diéthylèneglycol, les éthers de glycol, les alkyléthers de glycol ou de diéthylèneglycol.

Les polymères siliconés greffés selon l'invention peuvent être dissous dans ledit mileu cosmétiquement acceptable ou utilisés sous forme de dispersion aqueuse de particules.

La composition de l'invention peut également contenir au moins un additif choisi parmi les épaississants sans chaîne grasse, les esters d'acides gras, les esters d'acides gras et de glycérol, les silicones, les tensioactifs, les parfums, les conservateurs, les filtres solaires, les protéines, les vitamines, les polymères, les huiles végétales, animales, minérales ou synthétiques et tout autre additif classiquement utilisé dans le domaine cosmétique.

Ces additifs sont présents dans la composition selon l'invention dans des proportions pouvant aller de 0 à 20% en poids par rapport au poids total de la composition. La quantité précise de chaque additif est fonction de sa nature et est déterminée facilement par l'homme de l'art.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions selon l'invention peuvent se présenter sous forme de gel, de lait, de crème, de lotion plus ou moins épaissie ou de mousse.

Elles sont plus particulièrement des lotions de mise en plis, des lotions pour le brushing, des compositions de fixation (laques) et de coiffage. Les lotions peuvent être conditionnées sous diverses formes notamment dans des vaporisateurs, des flacons pompes ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour la fixation ou le traitement des cheveux.

Les compositions peuvent être également des shampooings, des compositions à rincer ou non, à appliquer avant ou après un shampooing, une coloration, une décoloration, une permanente ou un défrisage.

Lorsque la composition selon l'invention est conditionnée sous forme d'aérosol en vue d'obtenir une laque ou une mousse aérosol, elle comprend au moins un agent propulseur qui peut être choisi parmi les hydrocarbures volatils tels que le n-butane, le propane, l'isobutane, le pentane, un hydrocarbure chloré et/ou fluoré et leurs mélanges. On peut également utiliser en tant qu'agent propulseur le gaz carbonique, le protoxyde d'azote, le diméthyléther, l'azote, l'air comprimé et leurs mélanges.

L'invention a encore pour objet un procédé de traitement non-thérapeutique des matières kératiniques telles que les cheveux humains consistant à appliquer sur celles-ci une composition telle que définie précédemment puis à effectuer éventuellement un rinçage à l'eau.

L'invention va être maintenant plus complètement illustrée à l'aide des exemples suivants qui ne sauraient être considérés comme la limitant aux modes de réalisation décrits.

### EXEMPLES

### EXEMPLE 1 Gel de coiffage

- Polymère siliconé greffé de formule (I) de structure polyméthyl/méthylsiloxane à groupements propyl thio-3 acide polyméthacrylique et groupements propyl thio-3 poly(méthacrylate de méthyle) 1 g en MA
- Terpolymère acide méthacrylique/acrylate d'éthyle/méthacrylate de stéaryle oxyéthyléné (55/35/10) en dispersion aqueuse à 30% vendu sous le nom ACRYSOL 22 par la Société ROHM & HAAS 1 g en MA
- Aminométhylpropanol neutralisation à 100% dudit polymère siliconé et du terpolymère qsp
- Eau déminéralisée qsp 100 g

### EXEMPLE 2 Gel de coiffage

- Polymère siliconé greffé de formule (I) de structure polyméthyl/méthylsiloxane à groupements propyl thio-3 acide polyméthacrylique et groupements propyl thio-3 poly(méthacrylate de méthyle) 2 g en MA
- Hydroxyéthylcellulose modifiée par une chaîne cétyle vendue sous le nom NATROSOL PLUS GRADE 330 S par la société AQUALON 3 g en MA
- Aminométhylpropanol neutralisation à 100% dudit polymère siliconé greffé qsp
- Eau déminéralisée qsp 100 g

### ESSAIS COMPARATIFS DE VISCOSITE

On étudie les propriétés rhéologiques d'un agent épaississant classique du type homopolymère poly(acide acrylique) réticulé comme le produit SYNTHALEN K vendu par la société 3V dans une solution aqueuse contenant 1% en poids de cet épaississant et dans une solution aqueuse contenant 1% en poids de cet épaississant et 1% en poids de polymère siliconé greffé tel que celui décrit dans les exemples 1 et 2. On mesure les viscosités des solutions épaissies au moyen d'un appareil RHEOMAT180 équipé du système CONTRAVES TV.

On étudie les propriétés rhéolologiques d'un polymère Pᵢ amphiphile comportant une chaîne grasse et au moins un motif hydrophile selon l'invention dans une solution aqueuse contenant 1% en poids de ce polymère amphiphile épaississant et dans une solution aqueuse contenant 1% en poids de cet épaississant et 1% en poids de polymère siliconé greffé tel que celui décrit dans les exemples 1 et 2. Dans ce qui suit l'indice i est spécifique au polymère amphiphile étudié.

Toutes les solutions sont neutralisées à pH 7,5 par de l'aminométhylpropanol.

Les polymères amphiphiles selon l'invention qui ont été étudiés sont les suivants :
- P₁ : Terpolymère acide méthacrylique/acrylate d'éthyle/méthacrylate de stéaryle oxyéthyléné (55/35/10) en dispersion aqueuse à 30% vendu sous le nom ACRYSOL 22 par la Société ROHM & HAAS ;
- P₂ : Terpolymère acide méthacrylique/acrylate d'éthyle/stéaryl polyéthoxylé allyl éther SALCARE-SC 90 vendu par la société ALLIED COLLOIDS (stéaryl polyéthoxylé à 1O moles d'oxyde d'éthylène noté stéareth-10) ;
- P₃ : Hydroxyéthylcellulose modifiée par une chaîne cétyle vendue sous le nom NATROSOL PLUS GRADE 330 S par la société AQUALON.

| Les viscosités des solutions sont indiquées en centipoises dans le tableau suivant : Polymère étudié | Viscosité en cps de la solution aqueuse contenant 1% d'agent épaississant seul ou le polymère siliconé greffé seul de l'exemple 1 ou 2 | Viscosité en cps de la solution aqueuse contenant 1% en poids d'agent épaississant et 1% en poids de polymère greffé siliconé de l'exempte 1 ou 2 |
|---|---|---|
| Polymère siliconé greffé des exemples 1 et 2 | 2,5 | pas d'épaississant |
| Homopolymère poly(acide acrylique) réticulé | 7500 | 6500 |
| P₁ | 1700 | 6900 |
| P₂ | 875 | 1590 |
| P₃ | 360 | 700 |

On constate que l'homopolymère poly(acide acrylique) reticulé en association avec le polymère siliconé de coiffage de l'invention diminue la viscosité de la formulation tandis que les polymères P₁ amphiphiles épaississants comportant une chaîne grasse et au moins un motif hydrophile selon l'invention augmentent sensiblement les viscosités des solutions contenant le polymère greffé siliconé.

### ESSAIS COMPARATIFS SUR LES PROPRIETES COSMETIQUES

On effectue un test d'estimation sensorielle sur un panel de 5 personnes. On étudie comme critères cosmétiques le démêlage et la douceur au toucher après application sur des mèches de cheveux, mouillées et sensibilisées du type SA 20. On applique sur chacune de ces 5 personnes, sur des mèches préalablement lavées au shampooing, les trois solutions A, B et C suivantes à une dose de 0,5 g pour 5 g de mèche :
Solution A contenant 1% en poids du polymère siliconé greffé de l'exemple 1 ou 2
Solution B contenant 1% en poids du polymère siliconé greffé de l'exemple 1 ou 2 et 1% en poids de l'homopolymère poly(acide acrylique) réticulé SYNTHALEN K
Solution C contenant 1% en poids du polymère siliconé greffé de l'exemple 1 ou 2 et 1% en poids de polymère P₁ tel que décrit ci-dessus

Toutes les solutions sont neutralisées à pH 7,5 par de l'aminométhylpropanol.

Pour chaque critère cosmétique, les personnes testées attribuent une note d'appréciation de 0 à 5. Les résultats des tests sont résumés dans le tableau ci-dessous.

| **Solutions testées** | **Appréciation** **du démêlage** | **Appréciation** **de la douceur au toucher** |
|---|---|---|
| A | 2 | 1,5 |
| B | 2,5 | 2,5 |
| C | 3,5 | 3,5 |

Les 5 personnes interrogées ont estimé que la présence du polymère amphiphile épaississant à chaîne grasse et au moins un motif hydrophile selon l'invention dans la solution C contenant le polymère siliconé greffé améliorait la douceur des cheveux au toucher et le démêlage des cheveux par rapport à la solution A contenant le polymère siliconé greffé seul ou la solution B contenant ledit polymère siliconé greffé en association avec l'épaississant classique du type homopolymère poly(acide acrylique) réticulé.

## Revendications

1. Composition cosmétique ou dermatologique destinée au traitement des matières kératiniques, contenant, dans un milieu cosmétiquement ou dermatologiquement acceptable, au moins un polymère siliconé greffé, à squelette polysiloxanique greffé par des monomères organiques non-siliconés et au moins un polymère amphiphile comportant au moins une chaîne grasse et au moins un motif hydrophile, **caractérisée par le fait que**
le polymère siliconé greffé est choisi parmi les polymères siliconés comportant dans leur structure le motif de formule (I) suivant : 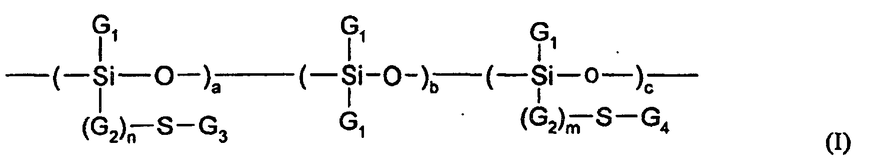 dans lequel les radicaux G₁, identiques ou différents, représentent l'hydrogène ou un radical alkyle en C₁-C₁₀ ou encore un radical phényle ; les radicaux G₂, identiques ou différents, représentent un groupe alkylène en C₁-C₁₀ ; G₃ représente un reste polymérique résultant de l'(homo)polymérisation d'au moins un monomère anionique à insaturation éthylénique ; G₄ représente un reste polymérique résultant de l'(homo)polymérisation d'au moins un monomère hydrophobe à insaturation éthylénique ; m et n sont égaux à 0 ou 1 ; a est un nombre entier allant de 0 et 50 ; b est un nombre entier pouvant être compris entre 10 et 350, c est un nombre entier allant de 0 et 50 ; sous réserve que l'un des paramètres a et c soit différent de 0 et les polymères amphiphiles comportant au moins une chaîne grasse et des motifs hydrophiles sont choisis dans le groupe constitué par :
(1) les holosides modifiés par des groupes comportant au moins une chaîne grasse
(2) les copolymères d'anhydride maléique ou de l'un de ses dérivés et de monomères comportant au moins une chaîne grasse:
(3) les polyuréthanes et leurs dérivés comportant des groupements comportant au moins une chaîne grasse ;
(4) les copolymères de l'acide crotonique et de monomères comportant au moins une chaîne grasse ;
(5) les copolymères de N-vinylpyrrolidone et de monomères comportant au moins une chaîne grasse ;
(6) les copolymères d'acide (méth)acrylique et de monomères comportant au moins une chaîne grasse ; ces monomères étant choisis dans le groupe constitué par les monomères hydrophobes à chaîne grasse, les monomères amphiphiles comportant une partie hydrophobe à chaîne grasse et une partie hydrophile ou bien leurs mélanges ;
(7) les copolymères non-ioniques de (méth)acrylate d'alkyle inférieur (C₁-C₆) et de monomères amphiphiles comportant une chaîne grasse ;
(8) les copolymères non-ioniques de (méth)acrylates hydrophiles et de monomères hydrophobes à chaîne grasse.

2. Composition selon la revendication 1, **caractérisée par le fait que** le motif de formule (I) présente au moins l'une des caractéristiques suivantes :
- les radicaux G₁ désignent un radical alkyle en C₁-C₁₀ ;
- n est non nul, et les radicaux G₂ représentent un radical divalent en C₁-C₃ ;
- G₃ représente un radical polymérique résultant de l'(homo)polymérisation d'au moins un monomère du type acide carboxylique à insaturation éthylénique ;
- G₄ représente un radical polymérique résultant de l'(homo)polymérisation d'au moins un monomère du type (méth)acrylate d'alkyle(C₁-C₁₀).

3. Composition selon la revendication 1 ou 2, **caractérisée par le fait que** le motif de formule (I) présente simultanément les caractéristiques suivantes :
- les radicaux G₁ désignent un radical méthyle ;
- n est non nul, et les radicaux G₂ représentent un radical propylène ;
- G₃ représente un radical polymérique résultant de l'(homo)polymérisation d'au moins l'acide acrylique et/ou l'acide méthacrylique ;
- G₄ représente un radical polymérique résultant de l'(homo)polymérisation d'au moins un monomère du type (méth)acrylate d'isobutyle ou de méthyle.

4. Composition selon l'une quelconque des revendications 1 à 3, **caractérisée par le fait que** la masse moléculaire en nombre du polymère siliconé greffé varie de 10 000 à 1 000 000 environ, et encore plus préférentiellement de 10 000 à 100 000 environ.

5. Composition selon l'une quelconque des revendications 1 à 4, **caractérisée par le fait que** le polymère siliconé greffé est utilisé en une quantité allant de 0,01 à 20% en poids par rapport au poids total de la composition et de préférence de 0,1 à 15% en poids et préférentiellement, cette quantité varie de 0,1 à 15% en poids et encore plus préférentiellement de 0,5 à 10% en poids.

6. Composition selon l'une quelconque des revendications 1 à 5, **caractérisée par le fait que** les polymères amphiphiles comportant au moins une chaîne grasse et au moins un motif hydrophile sont utilisés en une quantité allant de 0,01 à 10% en poids du poids total de la composition et plus préférentiellement de 0,5 à 10% en poids.

7. Composition selon l'une quelconque des revendications 1 à 6, **caractérisée par le fait qu'**elle contient en plus au moins un additif choisi dans le groupe constitué par les épaississants sans chaîne grasse, les esters d'acides gras, les esters d'acides gras et de glycérol, les silicones, les tensioactifs, les parfums, les conservateurs, les filtres solaires, les protéines, les vitamines, les polymères, les huiles végétales, animales, minérales ou synthétiques et tout autre additif classiquement utilisé dans le domaine cosmétique.

8. Composition selon l'une quelconque des revendications 1 à 7, **caractérisée par le fait que** le milieu cosmétiquement ou dermatologiquement acceptable est constitué par de l'eau ou un mélange d'eau et d'au moins un solvant cosmétiquement acceptable.

9. Composition selon la revendication 8, **caractérisée par le fait que** les solvants cosmétiquement acceptables sont choisis dans le groupe constitué par les monoalcools, les polyalcools, les éthers de glycol, les esters d'acides gras et leurs mélanges.

10. Composition selon l'une quelconque des revendications 1 à 9, **caractérisée par le fait que** le polymère siliconé greffé est dissous dans le milieu cosmétiquement ou dermatologiquement acceptable ou utilisé sous forme de dispersion aqueuse de particules.

11. Composition selon l'une quelconque des revendications 1 à 10, **caractérisée par le fait que** les matières kératiniques sont des cheveux humains.

12. Composition selon l'une quelconque des revendications 1 à 11, **caractérisée par le fait qu'**elle se présente sous forme de gel, de lait, de crème, de lotion plus ou moins épaissie ou de mousse.

13. Composition selon l'une quelconque des revendications 1 à 12, **caractérisée par le fait qu'**elle est un produit de coiffage.

14. Composition selon l'une quelconque des revendications 1 à 13**, caractérisée par le fait qu'**elle est un produit capillaire choisi dans le groupe constitué par des shampooings ; des produits capillaires à rincer ou non, à appliquer avant ou après un shampooing, une coloration, une décoloration, une permanente ou un défrisage.

15. Composition selon l'une quelconque des revendications 1 à 14, **caractérisée par le fait qu'**elle est conditionnée sous forme de vaporisateur, de flacon pompe ou bien dans un récipient aérosol en vue d'obtenir un spray, une laque ou une mousse.

16. Procédé non-thérapeutique de traitement des matières kératiniques, en particulier des cheveux humains, **caractérisé par le fait qu'**il consiste à appliquer sur lesdites matières une composition telle que définie selon l'une quelconque des revendications 1 à 15 puis à effectuer éventuellement un rinçage à l'eau.

## Patentansprüche

1. Kosmetische oder dermatologische Zusammensetzung, die zur Behandlung von Keratinsubstanzen vorgesehen ist und die in einem kosmetisch oder dermatologisch akzeptablen Medium mindestens ein gepfropftes Siliconpolymer mit Polysiloxangerüst, auf das nicht siliconhaltige, organische Monomere gepfropft sind, und mindestens ein amphiphiles Polymer, das mindestens eine Fettkette und mindestens eine hydrophile Einheit aufweist, enthält, **dadurch gekennzeichnet, dass** das gepfropfte Siliconpolymer unter den Siliconpolymeren ausgewählt ist, die in ihrer Struktur die Einheit der folgenden Formel (I) enthalten: 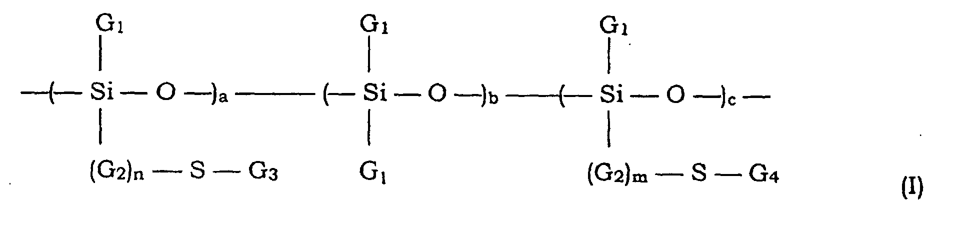 worin die Gruppen G₁, die identisch oder voneinander verschieden sind, Wasserstoff, C₁₋₁₀-Alkyl oder Phenyl; die Gruppen G₂, die identisch oder voneinander verschieden sind, C₁₋₁₀-Alkylen; G₃ eine Polymergruppe, die aus der (Homo)polymerisation mindestens eines anionischen Monomers mit ethylenischer Doppelbindung resultiert; G₄ eine Polymergruppe, die bei der (Homo)polymerisation mindestens eines hydrophoben Monomers mit ethylenischer Doppelbindung entsteht; m und n 0 oder 1; a 0 oder eine ganze Zahl im Bereich von 1 bis 50; b eine ganze Zahl im Bereich von 10 bis 350 und c 0 oder eine ganze Zahl von 1 bis 50 bedeuten, mit der Maßgabe, dass einer der Parameter a oder c von 0 verschieden ist, und die amphiphilen Polymere, die mindestens eine Fettkette und mindestens eine hydrophile Einheit enthalten, unter den folgenden Verbindungen ausgewählt sind:
(1)Holosiden, die mit Gruppen modifiziert sind, die mindestens eine Fettkette aufweisen;
(2) Copolymeren von Maleinsäureanhydrid oder einem seiner Derivate und Monomeren, die mindestens eine Fettkette aufweisen;
(3) Polyurethanen und deren Derivaten, die Gruppen tragen, die mindestens eine Fettkette aufweisen;
(4) Copolymeren von Crotonsäure und Monomeren, die mindestens eine Fettkette aufweisen;
(5) Copolymeren von N-Vinylpyrrolidon und Monomeren, die mindestens eine Fettkette aufweisen;
(6) Copolymeren von (Meth)acrylsäure und Monomeren, die mindestens eine Fettkette aufweisen, wobei diese Monomere unter den hydrophoben Monomeren mit Fettkette, den amphiphilen Monomeren, die einen hydrophoben Bereich mit Fettkette und einen hydrophilen Bereich enthalten, und deren Gemischen ausgewählt sind;
(7) nichtionischen Copolymeren von Alkyl(meth)acrylaten (niedere C₁₋₆-Alkylguppe) und amphiphilen Monomeren mit Fettkette;
(8) nichtionischen Copolymeren von hydrophilen (Meth)acrylaten und hydrophoben Monomeren mit Fettkette.

2. Zusammensetzung nach Anspruch 1, **dadurch gekennzeichnet, dass** die Einheit der Formel (I) mindestens eine der folgenden Eigenschaften aufweist:
- die Gruppen G₁ bedeuten eine C₁₋₁₀-Alkylgruppe;
- n ist nicht 0 und die Gruppen G₂ bedeuten eine zweiwertige Gruppe mit 1 bis 3 Kohlenstoffatomen;
- G₃ bedeutet eine Polymergruppe, die bei der (Homo)polymerisation mindestens eines Monomers vom Typ einer Carbonsäure mit ethylenischer Doppelbindung gebildet wird; und
- G₄ bedeutet eine Polymergruppe, die bei der (Homo)polymerisation mindestens eines Monomers vom Typ C₁₋₁₀-Alkyl(meth)acrylat entsteht.

3. Zusammensetzung nach Anspruch 1 oder 2, **dadurch gekennzeichnet, dass** die Einheit der Formel (I) alle folgenden Eigenschaften aufweist:
- die Gruppen G₁ bedeuten Methyl;
- n ist nicht 0 und die Gruppen G₂ bedeuten Propylen;
- G₃ bedeutet eine Polymergruppe, die bei der (Homo)polymerisation von zumindest Acrylsäure und/oder Methacrylsäure entsteht;
- G₄ bedeutet eine Polymergruppe, die bei der (Homo)polymerisation mindestens eines Monomers vom Typ Isobutyl(meth)acrylat oder Methyl(meth)acrylat entsteht.

4. Zusammensetzung nach einem der Ansprüche 1 bis 3, **dadurch gekennzeichnet, dass** die zahlenmittlere Molmasse des gepfropften Siliconpolymers im Bereich von etwa 10 000 bis 1 000 000 und noch bevorzugter im Bereich von etwa 10 000 bis 100 000 liegt.

5. Zusammensetzung nach einem der Ansprüche 1 bis 4, **dadurch gekennzeichnet, dass** das gepfropfte Siliconpolymer in einer Menge von 0,01 bis 20 Gew.-%, bezogen auf das Gesamtgewicht der Zusammensetzung, und vorzugsweise 0,1 bis 15 Gew.-% verwendet wird, wobei dieser Mengenanteil bevorzugt im Bereich von 0,1 bis 15 Gew.-% und noch bevorzugter im Bereich von 0,5 bis 10 Gew.-% liegt.

6. Zusammensetzung nach einem der Ansprüche 1 bis 5, **dadurch gekennzeichnet, dass** die amphiphilen Polymere, die mindestens eine Fettkette und mindestens eine hydrophile Einheit aufweisen, in einer Menge von 0,01 bis 10 Gew.-%, bezogen auf das Gesamtgewicht der Zusammensetzung, und noch bevorzugter 0,5 bis 10 Gew.-% verwendet werden.

7. Zusammensetzung nach einem der Ansprüche 1 bis 6, **dadurch gekennzeichnet, dass** sie ferner mindestens einen Zusatzstoff enthält, der unter den Verdickungsmitteln ohne Fettkette, Fettsäureestern, Estern von Fettsäuren und Glycerin, Siliconen, grenzflächenaktiven Stoffen, Parfums, Konservierungsmitteln, Sonnenschutzfiltern, Proteinen, Vitaminen, Polymeren, pflanzlichen Ölen, tierischen Ölen, Mineralölen oder synthetischen Ölen oder beliebigen anderen, herkömmlich in der Kosmetik verwendeten Zusatzstoffen ausgewählt ist.

8. Zusammensetzung nach einem der Ansprüche 1 bis 7, **dadurch gekennzeichnet, dass** das kosmetisch oder dermatologisch akzeptable Medium aus Wasser oder einem Gemisch von Wasser und mindestens einem kosmetisch akzeptablen Lösungsmittel besteht.

9. Zusammensetzung nach Anspruch 8, **dadurch gekennzeichnet, dass** die kosmetisch akzeptablen Lösungsmittel unter den Monoalkoholen, Polyalkoholen, Glykolethern, Fettsäureestern und deren Gemischen ausgewählt sind.

10. Zusammensetzung nach einem der Ansprüche 1 bis 9, **dadurch gekennzeichnet, dass** das gepfropfte Siliconpolymer in dem kosmetisch oder dermatologisch akzeptablen Medium gelöst ist oder in Form einer wässrigen Dispersion von Partikeln verwendet wird.

11. Zusammensetzung nach einem der Ansprüche 1 bis 10, **dadurch gekennzeichnet, dass** es sich bei den Keratinsubstanzen um menschliches Haar handelt.

12. Zusammensetzung nach einem der Ansprüche 1 bis 11, **dadurch gekennzeichnet, dass** sie als Gel, Milch, Creme, mehr oder weniger dickflüssige Lotion oder Schaum vorliegt.

13. Zusammensetzung nach einem der Ansprüche 1 bis 12, **dadurch gekennzeichnet, dass** es sich um ein Produkt für die Frisurengestaltung handelt.

14. Zusammensetzung nach einem der Ansprüche 1 bis 13, **dadurch gekennzeichnet, dass** es sich um ein Produkt für die Haarbehandlung handelt, das unter den Haarwaschmitteln und Produkten für das Haar, die ausgespült oder nicht ausgespült werden, zur Anwendung vor oder nach einer Haarwäsche, Färbung, Entfärbung, permanenten Verformung oder Entkräuselung ausgewählt ist.

15. Zusammensetzung nach einem der Ansprüche 1 bis 14, **dadurch gekennzeichnet, dass** sie zur Bildung eines Sprays, eines Lacks oder eines Schaums in einem Zerstäuber, Pumpflakon oder Aerosolbehälter konfektioniert ist.

16. Nicht therapeutisches Verfahren zur Behandlung von Keratinsubstanzen und insbesondere menschlichem Haar, **dadurch gekennzeichnet, dass** es darin besteht, auf die Keratinsubstanzen eine Zusammensetzung nach einem der Ansprüche 1 bis 15 aufzutragen und anschließend gegebenenfalls mit Wasser zu spülen.

## Claims

1. Cosmetic or dermatological composition intended for treating keratin substances, **characterized in that** it contains, in a cosmetically or dermatologically acceptable medium, at least one grafted silicone polymer containing a polysiloxane skeleton grafted with non-silicone organic monomers and at least one amphiphilic polymer containing at least one fatty chain and at least one hydrophilic unit, **characterized in that** the grafted silicone polymer is chosen from silicone polymers comprising in their structure the unit of formula (I) below: 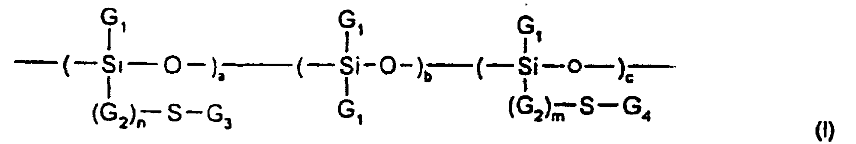 in which the radicals G₁, which may be identical or different, represent hydrogen or a C₁-C₁₀ alkyl radical or alternatively a phenyl radical; the radicals G₂, which may be identical or different, represent a C₁-C₁₀ alkylene group; G₃ represents a polymer residue resulting from the (homo)polymerization of at least one anionic monomer containing ethylenic unsaturation; G₄ represents a polymer residue resulting from the (homo)polymerization of at least one hydrophobic monomer containing ethylenic unsaturation; m and n are equal to 0 or 1; a is an integer ranging from 0 to 50; b is an integer which may be between 10 and 350; c is an integer ranging from 0 to 50; with the proviso that one of the parameters a and c is other than 0, and the amphiphilic polymers comprising at least one fatty chain and hydrophilic units are chosen from the group consisting of:
(1) holosides modified with groups comprising at least one fatty chain;
(2) copolymers of maleic anhydride or of a derivative thereof and of monomers comprising at least one fatty chain;
(3) polyurethanes and derivatives thereof comprising groups comprising at least one fatty chain;
(4) copolymers of crotonic acid and of monomers comprising at least one fatty chain;
(5) copolymers of N-vinylpyrrolidone and of monomers comprising at least one fatty chain;
(6) copolymers of (meth)acrylic acid and of monomers comprising at least one fatty chain; these monomers being chosen from the group consisting of fatty-chain hydrophobic monomers, amphiphilic monomers comprising a fatty-chain hydrophobic portion and a hydrophilic portion, or mixtures thereof;
(7) nonionic copolymers of (C₁-C₆) lower alkyl (meth)acrylate and of amphiphilic monomers comprising a fatty chain;
(8) nonionic copolymers of hydrophilic (meth)acrylates and of fatty-chain hydrophobic monomers.

2. Composition according to Claim 1, **characterized in that** the unit of formula (I) has at least one of the following characteristics:
- the radicals G₁ denote a C₁-C₁₀ alkyl radical;
- n is non-zero and the radicals G₂ represent a divalent C₁-C₃ radical;
- G₃ represents a polymeric radical resulting from the (homo)polymerization of at least one monomer of the carboxylic acid type containing ethylenic unsaturation;
- G₄ represents a polymeric radical resulting from the (homo)polymerization of at least one monomer of the (C₁-C₁₀) alkyl (meth)acrylate type.

3. Composition according to Claim 1 or 2, **characterized in that** the unit of formula (I) simultaneously has the following characteristics:
- the radicals G₁ denote a methyl radical;
- n is non-zero and the radicals G₂ represent a propylene radical;
- G₃ represents a polymeric radical resulting from the (homo)polymerization of at least one acrylic acid and/or methacrylic acid;
- G₄ represents a polymeric radical resulting from the (homo)polymerization of at least one monomer of the isobutyl (meth)acrylate or methyl (meth)acrylate type.

4. Composition according to any one of Claims 1 to 3, **characterized in that** the number-average molecular mass of the grafted silicone polymer ranges approximately from 10,000 to 1,000,000 and even more preferably approximately from 10,000 to 100,000.

5. Composition according to any one of Claims 1 to 4, **characterized in that** the grafted silicone polymer is used in an amount ranging from 0.01 to 20% by weight relative to the total weight of the composition and preferably from 0.1 to 15% by weight and even more preferably from 0.5 to 10% by weight.

6. Composition according to any one of Claims 1 to 5, **characterized in that** the amphiphilic polymers comprising at least one fatty chain and at least one hydrophilic unit are used in an amount ranging from 0.01 to 10% by weight relative to the total weight of the composition, and more preferably from 0.5 to 10% by weight.

7. Composition according to any one of Claims 1 to 6, **characterized in that** it also contains at least one additive chosen from the group consisting of fatty chain-free thickeners, fatty acid esters, fatty acid esters of glycerol, silicones, surfactants, fragrances, preserving agents, sunscreens, proteins, vitamins, polymers, plant, animal, mineral or synthetic oils or any other additive conventionally used in the cosmetic field.

8. Composition according to any one of Claims 1 to 7, **characterized in that** the cosmetically or dermatologically acceptable medium consists of water or a mixture of water and at least one cosmetically acceptable solvent.

9. Composition according to Claim 8, **characterized in that** the cosmetically acceptable solvents are chosen from the group consisting of monoalcohols, polyalcohols, glycol ethers and fatty acid esters, and mixtures thereof.

10. Composition according to any one of Claims 1 to 9, **characterized in that** the grafted silicone polymer is dissolved in the cosmetically or dermatologically acceptable medium or used in the form of an aqueous dispersion of particles.

11. Composition according to any one of Claims 1 to 10, **characterized in that** the keratin substance is human hair.

12. Composition according to any one of Claims 1 to 11, **characterized in that** it is in the form of a gel, a milk, a cream, a more or less thickened lotion or a foam.

13. Composition according to any one of Claims 1 to 12, **characterized in that** it is a styling product.

14. Composition according to any one of Claims 1 to 13, **characterized in that** it is a hair product chosen from the group consisting of shampoos and rinse-out or leave-in hair products, to be applied before or after shampooing, dyeing, bleaching, permanent-waving or straightening the hair.

15. Composition according to any one of Claims 1 to 14, **characterized in that** it is packaged in the form of a vaporizer, a pump-dispenser bottle or in an aerosol container in order to obtain a spray, a lacquer or a foam.

16. Non-therapeutic process for treating keratin substances, in particular human hair, **characterized in that** it consists in applying a composition as defined according to any one of Claims 1 to 15 to the said substances, and then optionally in rinsing with water.
